# EUROPEAN PATENT SPECIFICATION

(11) **EP 0 645 995 B1**
(45) Date of publication and mention of the grant of the patent: **13.01.1999**
(21) Application number: 93916626.0
(22) Date of filing: 18.06.1993
(51) Int. Cl.: A61F 13/02, A61F 7/00

(54) **WOUND COVERING**
WUNDABDECKUNG
PANSEMENT POUR PLAIE

(30) Priority: 19.06.1992 US 900656
(43) Date of publication of application: 05.04.1995
(60) Divisional application: 96113798.1
(73) Proprietor: AUGUSTINE, Scott D., Bloomington, MN 55438 (US)
(72) Inventor: AUGUSTINE, Scott D., Bloomington, MN 55438 (US)
(74) Representative: Plougmann, Vingtoft & Partners A/S
(86) International application number: US9305876
(87) International publication number: WO9400090

(56) References cited:
- EP-A- 0 424 165
- EP-A- 0 485 657
- DE-A- 3 118 232
- DE-A- 3 539 533
- FR-A- 1 527 887
- FR-A- 2 544 202
- GB-A- 2 199 501
- US-A- 3 610 238
- US-A- 4 540 412
- US-A- 4 572 188
- US-A- 4 641 643
- US-A- 4 969 881

## Description

### BACKGROUND OF THE INVENTION

### 1.Field of the Invention

This invention relates to a wound covering for wound treatment. The wound covering overlays the wound area without touching the wound itself. The wound covering preferably controls the temperature, humidity and other aspects of the environment at the wound site.

### 2.Technical Background

Traditional wound coverings such as bandages are used to mechanically close wounds. Such bandages typically cover and touch the wound. Bandage contact with the wound and can interfere with the healing process.

The benefits of application of heat to a wound are known and documented benefits include: increased cutaneous and subcutaneous blood flow; increased partial pressure of oxygen at the wound site; increased immune system functions, including increased migration of white blood cells to the site.

However, in modern times, heat therapy for the treatment of wounds and infection has been difficult to achieve in practice. Additionally the availability of antibiotics have taken precedence over other therapies for the treatment of wounds and topical infections.

The benefits of controlling other environmental parameters around the wound site are not as well known. Controlling the humidity at the wound site as well as the benefits of isolating the wound have not been extensively studied and documented.

US 4,641,643 discloses a disposable release bandage comprising a flexible body, a flexible layer, a cover, an adhesive layer for affixing the flexible body to a patient's skin, a means for affixing the flexible layer to the flexible body, and a means for affixing the cover to the flexible layer.

EP-A-0 485 657 discloses a wound dressing having a foam layer onto which adhesives are placed on both major sides of a foam layer, then each layer of adhesive is covered with release liners.

DE-A-35 39 533 discloses a wound dressing or bandage that has at least one component which is in direct contact with the wound.

EP-A-0 421 165 discloses a wound dressing containing a hydrogel material within a cavity in a transparent layer, the transparent layer optionally being printed with a grid layer.

### SUMMARY OF THE INVENTION

The wound covering includes a peripheral sealing ring which, in use, completely surrounds the area of the wound. The upper surface of the peripheral sealing ring is spanned by a continuous barrier layer which is preferably transparent and substantially impermeable. An adhesive and a suitable release liner is applied to the lower surface of the peripheral sealing ring to facilitate the application of the wound covering to the patient's skin. Once in position, the sealing ring and the barrier layer define a wound treatment volume which surrounds the wound.

In accordance with actively heated embodiments of the invention, the barrier layer may include a pocket adapted to receive an active heater. An alternate form of the invention provides for the transport of heated air from a remote source, to the wound treatment volume. In the active heater embodiments a thermostat and/or a pressure activated switch may be used to control the heating effects of the electrically powered heater. Passively heated embodiments are contemplated as well. These passive versions of the device include the use of thermally insulating coverings which retain body heat within the treatment volume. These reflectors or insulators may be placed in a pocket formed in the barrier layer. Each of these heated embodiments promote wound healing by maintaining the wound site at a generally elevated but controlled temperature.

In general the peripheral sealing ring is made from an absorbent material which may act as a reservoir to retain and dispense moisture into the treatment volume increasing the humidity at the wound site. The reservoir may also contain and deliver drugs and the like to promote healing.

### BRIEF DESCRIPTION OF THE DRAWINGS

Illustrative but not limiting embodiments of the invention are shown in the attached drawings. Throughout the several figures like reference numerals refer to identical structure throughout, in which:
Fig. 1A is an exploded view of the wound covering;
Fig. 1B illustrates an assembled view of the wound covering of Fig. 1A;
Figs. 2A and 2B is a view of an alternate wound covering;
Fig. 3A is an exploded view of an alternate wound covering;
Fig. 3B is an assembly view of the wound covering of Fig. 3A;
FIG.4 is a side elevation view of a wound covering;
FIG.5 is an enlarged top plan view of a wound covering;
FIG.6 is an enlarged sectional view taken along line A-A of FIG.5;
FIG.7 is a bottom view of the wound covering of FIG.4;
Fig. 8A is an exploded view of an alternate wound covering:
Fig. 8B is an assembly view showing the air flow through the wound covering;
Fig. 9A is a perspective view of an alternate wound covering;
Fig. 9B is a side view of the wound covering of Fig. 9A;
Fig. 10 is a perspective view of an alternate wound covering;
Fig. 11A is a perspective view of an alternate wound covering;
Fig. 11B is a side cross-sectional view of the wound covering of Fig. 11A;
Fig. 11C is a view of the wound covering of Fig. 11A;
Fig. 12 is a perspective view of an alternate connector apparatus for the wound covering;
Fig. 13A is an alternate connector arrangement for the wound covering;
Fig. 13B is a side sectional view of the wound covering of 13A;
Fig. 14 is a view of a rigid connector for engagement with a wound covering;
Fig. 15 is an alternate fluid inlet line for the wound covering;
Fig. 16A is a view of a two ply barrier layer wound covering;
Fig. 16B is a cross-sectional view of the wound covering of 16A;
Fig. 17 is an alternate wound covering;
Fig. 18A is an alternate wound covering;
Fig. 18B is a side sectional view of the wound covering of Fig. 18A; and
Fig. 19 is a view of an alternate wound covering.

### DETAILED DESCRIPTION OF THE PREFERRED EMBODIMENTS

The present invention is directed to a non-contact wound covering for controlling the local environment at a wound site on a patient. The wound covering protects the wound from contamination by materials from the outside environment and also prevents the wound site from shedding contaminants into the local environment of the patient, i.e. the hospital room. The treatment volume formed over the wound site can be controlled to create an optimal healing environment. The word "wound" as used herein refers generically to surgical incisions, ulcers, or other lesions or breaks in the skin.

Each embodiment of the wound covering includes three basic elements. First a vertical wall is provided to encircle the wound area on the surface of the patient's skin. This vertical structure is self supporting and provides an upper surface to support a barrier layer above the level of the wound. This structure is referred to throughout as the peripheral sealing ring. The next element is a barrier layer which is attached to the peripheral sealing ring. Together these elements form an enclosure or wound treatment volume over the wound site. The fact that the barrier layer does not contact the wound itself promotes healing by minimizing mechanical stresses on the tissues. The barrier layer spans the entire wound area and attaches to the peripheral sealing ring. The third element is an adhesive and a complimentary release liner assembly which is attached to the lower surface of the sealing ring to facilitate attachment of the wound covering to the skin of the patient. As will be discussed in the various examples and illustrations detailed below, the three basic components of the wound covering are combined with other elements to provide an optimal healing environment at the wound site.

In accordance with the invention the climate within the wound treatment volume may be controlled. Typically the temperature, humidity, and gas composition is controlled. Also aerosolized medications or compounds can be released into this volume as well. The above list is exemplary of the climate controls which may promote healing of the wound, and is not intended to limit the scope of the present invention. It will be understood by those skilled in the art that numerous other climate factors can be controlled within the treatment volume of the present wound covering system without departing from the scope of the invention.

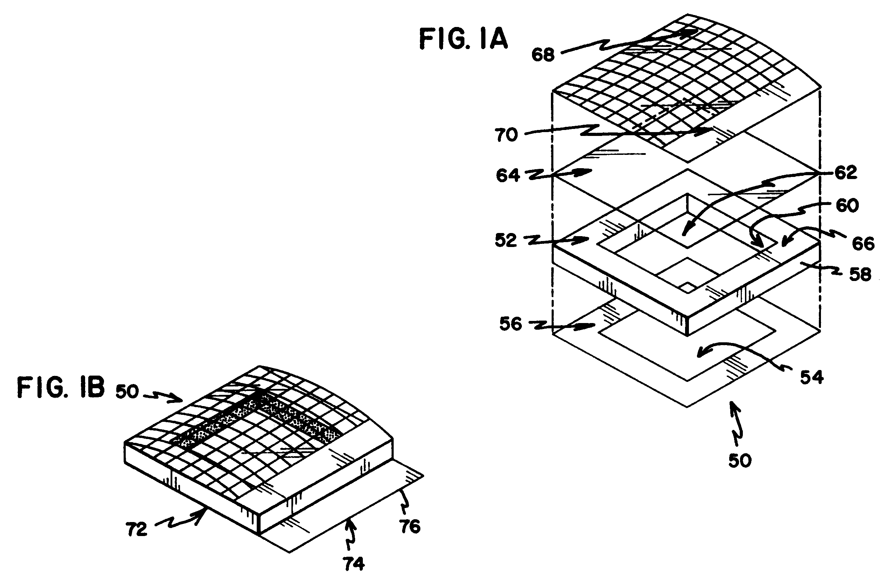

Fig. 1A illustrates an exploded view of the wound covering 50. In this embodiment the peripheral sealing ring 52 is substantially square in outline. The peripheral sealing ring 52 is intended to be attached to uninjured skin surrounding the wound area 54 using an adhesive 56. In this embodiment a layer of adhesive hydrogel is shown as the adhesive 56. In this embodiment the peripheral sealing ring 52 is preferably constructed of an open cell hydrophilic foam plastic having a sealed outer surface 58 which isolates the wound from the environment. The peripheral sealing ring is fabricated from a material which is stiff but which may conform to the curved surface of the patient's body. The inner surface 60 of the sealing ring 52 is preferably porous or absorbent so that it can form a reservoir to contain and release moisture or water vapor into the air within the treatment volume 62 to create a high humidity environment if desired. Additionally, the hydrophilic absorbent nature of the peripheral sealing ring 52 absorbs fluids and blood weeping from the wound.

A barrier layer 64 is preferably attached to the upper surface 66 of the peripheral sealing ring 52 to seal the treatment volume 62. The barrier layer 64 is preferably constructed of a clear flexible plastic film, such as polyethylene or polyvinylchloride. In this embodiment a wound tracing grid 68, also constructed of a clear flexible material, may optionally be attached to the barrier layer 64 so that the physician can draw the wound as an aid to track the healing process of the wound. The wound tracing grid preferably contains a labeling area 70 for identifying the patient, date when the wound was traced, and other patient medical data.

It will be understood by those skilled in the art that the volume of the peripheral sealing ring 52 will depend on the structural strength of the support material and the amount of fluid absorption desired. Additionally, the total area of the peripheral sealing ring 52 is dependent on the size of the wound. For example, larger wounds and more flexible covers will require a thicker sealing ring so that the center of the cover does not touch the wound.

The upper surface 66 of the peripheral sealing ring 52 is preferably sealed by extending the barrier layer 64 over the entire upper surface 66 as seen in the drawing. The adhesive 56 for attaching the peripheral sealing ring 52 to the wound area 54 may take any form. However, the preferred adhesive is a preferably a two-faced hydrogel which attaches to the lower surface 72 of the peripheral sealing ring 52. This adhesive 56 permits the attachment of the peripheral sealing ring 52 to the patient's skin. Finally, the peripheral sealing ring 52 may serve as a reservoir for retaining water or medicaments in the treatment volume 62 in order to maintain a high humidity in the air within the volume. Water may be added to the peripheral sealing ring 52 at any time during treatment.

It will be understood by those skilled in the art that the peripheral sealing ring 52 can be supplied in a variety of shapes and sizes to accommodate various wounds. The shapes may include circles, squares, or rectangles. Although it is preferred to dispense the wound covering as a unitary assembly, it should be apparent that individual segments of peripheral ring material could be assembled into any shape necessary to form a perimeter around the wound area. Likewise, the barrier layer 64 and wound tracing grid 68 could be provided in large sheets which may be cut to size and then attached to the peripheral sealing ring.

Figure 1B is an assembled view of the wound covering 50 of Fig. 1A. To dispense the assembled product, a release liner 74 is applied to the adhesive 56. The release liner may span the entire lower surface of the covering to maintains the sterility of the treatment volume 62. The release liner 74 preferably has a grip tab 76 to facilitate removal of the liner 74 from the wound covering 50 immediately prior to application of the wound covering 50 to the patient.

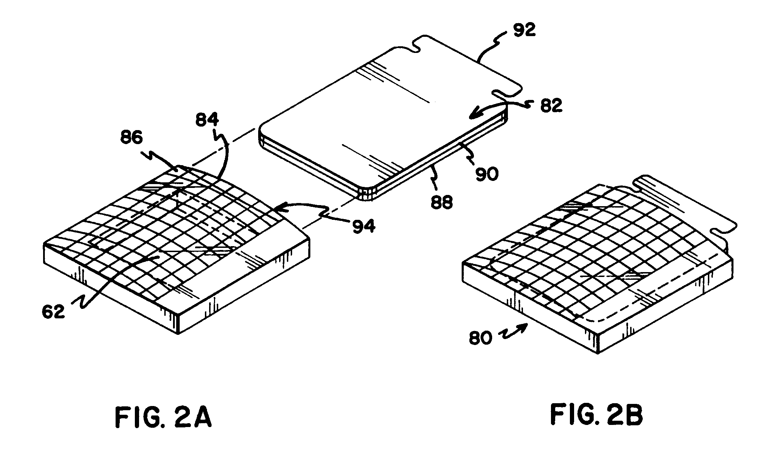

Figures 2A and 2B illustrate an alternate embodiment of the wound covering 80 utilizing passive heating of the treatment volume 62. Because heat is constantly being radiated from the body surface, the insulation properties of the trapped air within the treatment volume 62 will reduce this heat loss. By adding an infrared reflector 82 over the treatment volume 62, the infrared heat from the body can be reflected back to the skin for added passive heating.

One edge 84 of the wound tracing grid 86 is preferably not attached to the barrier layer to form an envelope or pocket 94 between the wound tracing grid 86 and the barrier layer. A piece of reflective foil material 88 may be inserted into the pocket 94. A thin layer of insulating material 90 may optionally be attached to the foil layer 88 to enhance heat retention and to provide the foil layer 88 with additional resiliency. A tab 92 is preferably attached to the infrared reflector 82 to allow easy insertion and removal from the pocket 94 and the wound covering 80.

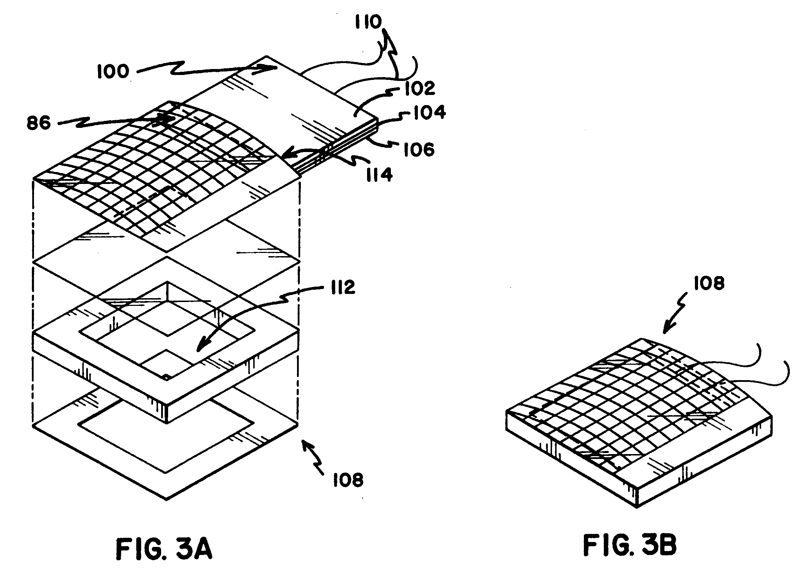

Figures 3A and 3B illustrate an alternate embodiment of a non-contact wound covering 108 utilizing active heating of the treatment volume 112. Small to medium sized wounds (up to approximately six inches in diameter) may be safely and easily heated utilizing the foil heater assembly 100. The heater assembly 100 preferably comprises a pressure-sensitive switch 102, an insulating layer 104, and a foil heater element 106.

The pressure-sensitive switch 102 is preferably laminated to the upper layer of the heater assembly 100. The purpose of the switch 102 is to shut off power to the heater element 106 in the event that external pressure is applied to the wound covering 108 with sufficient force to cause the heater element 106 to contact the skin or wound below. This is an important feature to prevent the possibility of applying heat and pressure to the skin at the same time. The combination of heat and pressure is known to cause burns even at low temperatures (40°C) because the pressure prevents blood flow in the skin making it susceptible to thermal injury. The pressure-sensitive switch 102 preferably covers the whole heater assembly 100 so that pressure applied anywhere to the surface of the heater assembly 100 will deactivate the heater element 106.

It will be understood that a variety of devices are suitable for use as the pressure-sensitive switch 102. For example, force sensing resistors resemble a membrane switch which changes resistance inversely with applied force. Devices of this type offer the substantial advantage of being low cost, flexible, and durable. It will be understood by those skilled in the art that a variety of other force sensing switch devices may be utilized as well.

The heater element 106 is preferably a thin film type resistance heater which is commercially available. Such thin film resistance heaters utilize low voltage, minimizing the electrical risk to the patient and allowing for battery-powered mobility. The heater element 106 is preferably sized for each wound covering 108. In actual use, the foil heater element 106 is preferably provided in large sheets with a pair of electrical leads 110 along one edge.

The foil heater assembly 100 is preferably inserted into a pocket 114 formed between the wound tracing grid 86 and the barrier layer as discussed above. Finally, a temperature monitoring device, such as a liquid crystal temperature monitor, may be applied to an upper surface of the foil heater assembly 100 or within the treatment volume 112 to monitor the temperature within the treatment volume 112.

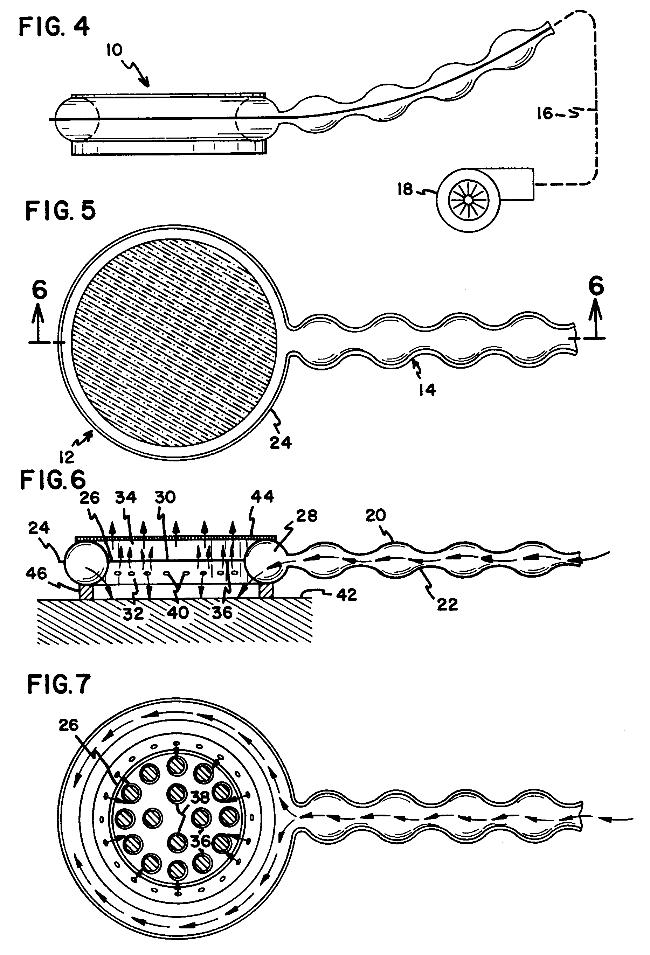

FIG.4 and FIG.5, illustrate an alternate embodiment, of the wound covering 10. In this embodiment the wound covering 10, includes a generally circular head, designated generally at 12, which transitions to an elongated non-kinking, collapsible air supply or hose 14.

The apparatus, as illustrated in FIG.4, is connected by suitable supply line or tube 16 to a source 18 of thermally controlled air which is schematically illustrated. The term air as used herein is intended to encompass mixtures of gases of controlled composition. The apparatus is constructed to apply a continuous stream of thermally controlled air to a wound treatment volume. While the apparatus was conceived and constructed for applying a heated stream of air, it may also be used to apply a cooled stream of air if required.

The specific form of the apparatus and details of construction can best be understood by reference to the various figures. The overall appearance of the wound covering is best seen in Fig. 4 and Fig. 5. It is preferred to construct the apparatus from top and bottom sheets of thin heat-sealable polymer film which overlay one another. A top sheet or membrane 20 overlies a bottom sheet or membrane 22 and they are heat sealed together along a plurality of seal lines, including a continuous outer seam 24, which extends in a circle around the head 12 and continues in a sinusoidal or convoluted fashion along and forming the air tube portion 14. An inner continuous circular seam 26 is provided as best seen in Fig. 6 and in Fig. 7. This inner seam secures the sheets together along a continuous circle to form the inner wall of a torus defining a supply volume 28.

The inner circular portion of the two sheets lying in the plane within the center of the supply volume 28 forms a wall 30 separating a lower wound treatment volume 32, from an upper insulation chamber 34. The wall 30 includes multiple apertures 36 formed by making small circular seals 38 and cutting and removing circular portions within the circular seals 38. Thus, a wall 30 with a plurality of apertures 36 is formed between the wound treatment volume 32 and insulation chamber 34. A plurality of apertures 40 are formed in the common circular wall surrounding the treatment volume 32 for distributing and conveying heated air or gases from the supply volume 28 into the wound treatment volume 32.

The heated air flowing into the treatment volume 32 bathes and contacts the wound surface of a patient's body 42. The air circulates throughout the wound treatment volume 32, and then passes through the apertures 36 into the upper or insulating chamber 34, where it then passes through a circular filter 44 forming an outer wall of the insulation chamber 34. The filter 44 filters the air leaving the wound treatment volume to trap contaminants shed from the wound. The filter 44 may be constructed of a filter paper bonded along its periphery to the outer tangential walls of the housing forming the torus or supply chamber 28. The filter paper also provides an insulating layer which suppresses loss of heat by radiation through the upper wall 30.

The lower surface of the head 12 as shown in Fig. 6 and Fig. 7, is preferably provided with a peripheral sealing ring 46 made of an absorbent material such as foam and bonded by suitable adhesive to the walls of the housing and the skin of the patient around the wound. Preferably, the foam or cotton peripheral sealing ring 46 is provided with a peel-off tape so that it adheres to the wall of the housing and on the other side to the skin of the patient. The adhesive or tape holds the apparatus in place and prevents airflow escape between the device and the skin of the patient. The absorbent material of the ring absorbs weeping blood and fluids and insulates the skin from direct heat in the tube.

The supply hose 14 is designed to be non-kinking by forming it of symmetrically convoluted flexible material. The hose and housing are integrally formed essentially of a unitary structure, such as a thin film membrane. The supply hose section 14 is inflatable upon the application of heated air through the supply line 16. The indentations in the hose section 14 permit it to bend without kinking and, thus, differentiate from a straight tubular hose which may kink when bent.

Since the thermal body treatment apparatus of the invention and the supply hose section are formed from two, thin, sealed-together membranes, the hose, and in fact the entire apparatus, is collapsible. This prevents the possibility of applying heat and pressure to the skin as might happen if a disoriented patient rolled over on the device. Instead, the weight of the patient's body would collapse the device, obstructing the flow of air, and preventing the application of heat.

The film membrane may preferably be transparent to enable viewing the wound without removal. However, for cosmetic reasons the barrier layer may be opaque. The filter paper 44 is attached across the tangential surfaces of the toroidal housing, thus providing a large area of filter for the escaping air. The head of the apparatus may be about one foot in diameter for most applications. However, it may be made smaller for certain other applications.

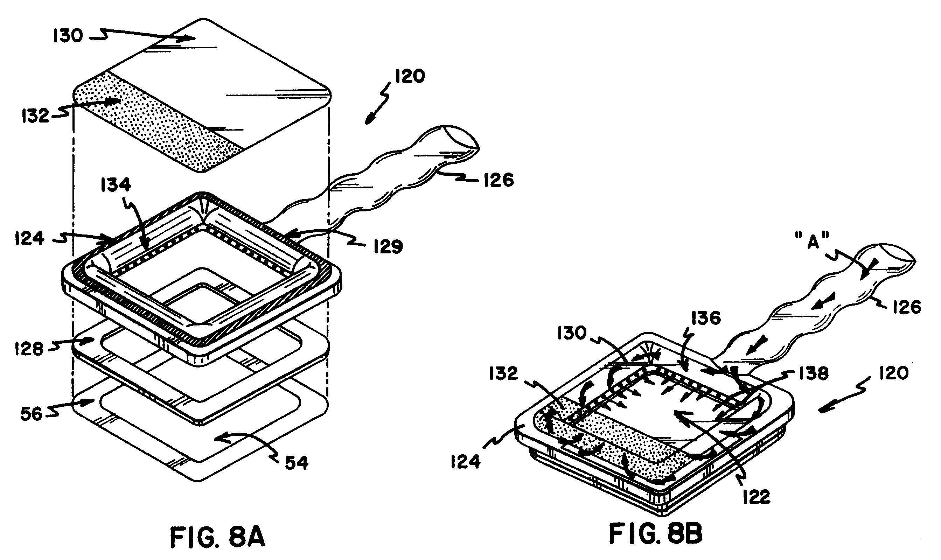

Fig. 8A illustrates an exploded view of an alternate embodiment of a non-contact wound covering 120 with climate control within the treatment volume 122. An inflatable structure 124 is preferably attached to a fluid inlet line 126 at a fluid inlet port 129 on the perimeter of the inflatable structure 124. The inflatable structure 124 is preferably attached to an absorbent peripheral sealing ring 128, which is in turn attached to the wound area 54 by a suitable adhesive 56. The peripheral sealing ring 128 preferably has a sealed outer surface and a porous inner surface which performs the same function as the peripheral sealing ring 52 discussed above. A barrier layer 130 having an exhaust filter 132 is attached to top surface 134 on the inflatable structure 124.

Turning now to the assembly illustrated in Fig. 8B, a gas illustrated by the arrows A, is introduced into the inflatable structure 124 from an external source (not shown) through the inlet line 126. The gas pressurizes the inflatable structure 124 in order to maintain the barrier layer 130 and exhaust filter 132 in an elevated position relative to the wound area 54. The inner surface 136 of the inflatable structure 124 preferably has a plurality of apertures 138 through which the fluid is introduced into the wound treatment volume 122. As the pressure within the treatment chamber increases, excess pressure is relieved through the exhaust filter 132. In this fashion, various fluids or gases can be introduced into the wound treatment volume 122.

The use of the term "fluid" in the context of this application refers to both liquid and gaseous materials, and combinations thereof. In one embodiment, oxygen may be introduced into the treatment volume 122 through the apertures 138 of the inflatable structure 124. The presence of oxygen within the wound treatment volume 122 may increase the oxygen available to the superficial layer of growing cells in the wound area 54. Nitric oxide may alternatively be infused into the treatment volume 122. Nitric oxide (NO) is a potent vasodilator which in theory may be absorbed across the wound surface and increase localized blood flow. A very small concentration of NO (parts per million) may provide this effect. NO may also be pre-absorbed into the absorbent peripheral sealing ring 128 and then allowed to passively diffuse into the volume once it is applied to the wound. Finally, gaseous or aerosolized medications or compounds may be introduced into the gas flow entering the treatment volume 122.

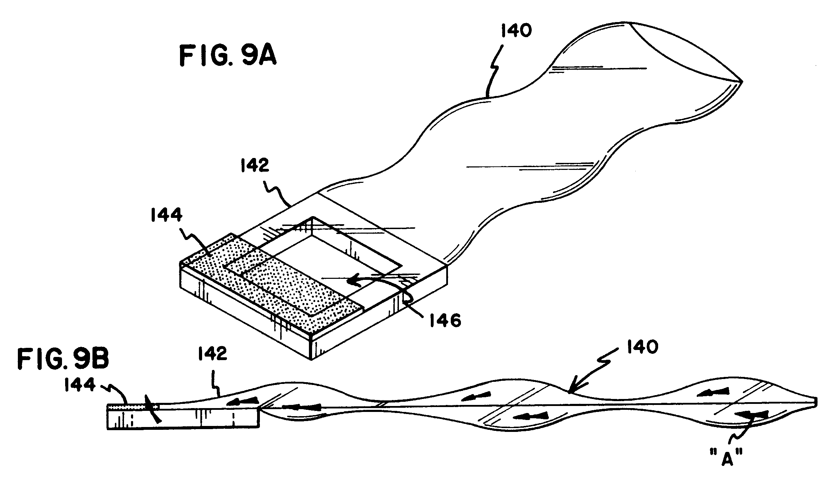

Fig. 9A and Fig. 9B illustrate an alternate embodiment of the climate control system discussed above wherein a fluid inlet line 140 may form part of a barrier layer 142. The barrier layer 142 is unitary with the fluid inlet line 140 and is preferably attached to an exhaust filter media 144 to allow excess pressure to be released from the wound treatment volume 146. In this embodiment, the filter media 144 forms part of the barrier 142. The arrows "A" in Figure 9B illustrate the movement of the fluid though the fluid inlet line 140, the treatment volume 146, and the exhaust filter 144.

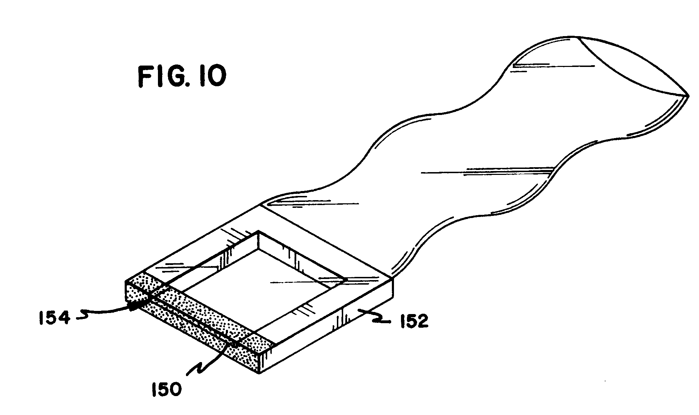

Fig. 10 illustrates an alternate embodiment wherein an exhaust filter 154 is retained in a recess 150 formed in one side of a peripheral sealing ring 152. This structure allows the excess fluid to be exhausted through the side of the peripheral sealing ring 152, rather than through the top, as illustrated in Fig. 9A and 9B.

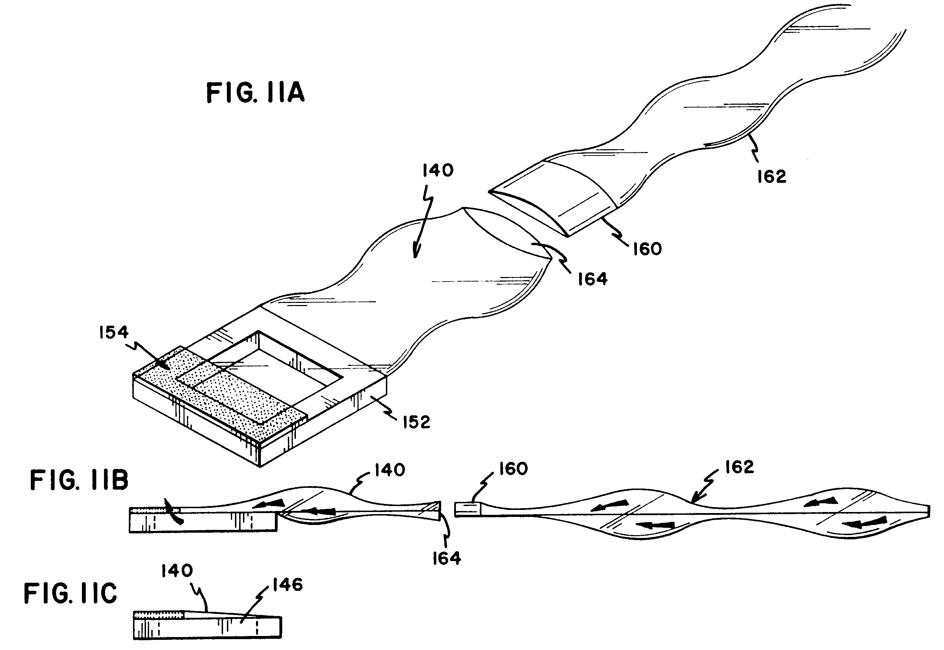

Fig. 11A is a perspective view of the embodiment illustrated in Fig. 9A wherein a connector 160 on the end of a fluid supply line 162 engages with an opening 164 on the fluid inlet line 140. Fig. 11B illustrates a side view of the fluid supply line 162 as it engages with the fluid inlet line 140. Fig. 11C illustrates the embodiment of 11A and 11B where the fluid inlet line 140 is folded over the top of the peripheral sealing ring 152 to seal the treatment volume 146 when the supply line 162 is uncoupled.

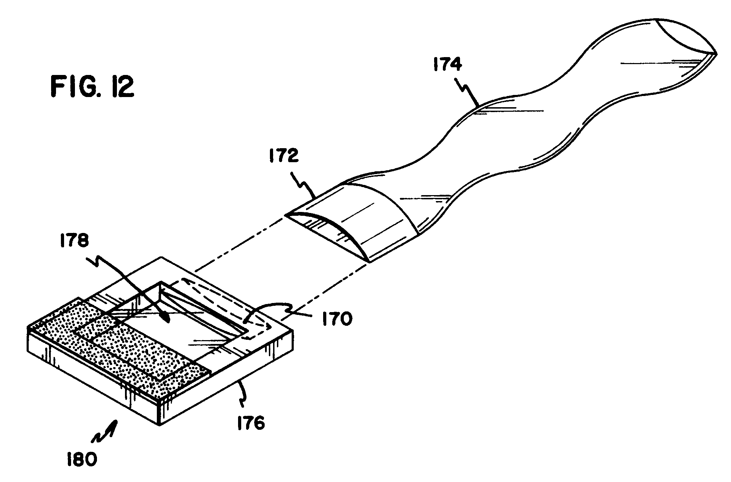

Fig. 12 illustrates an alternate embodiment in which a fluid inlet slot 170 engages with a rigid connector 172 on a fluid inlet line 174. The fluid inlet slot 170 forms an opening in one portion of the peripheral sealing ring 176. The opening is in fluid communication with the treatment volume 178. This configuration allows for quick disconnect of the fluid inlet line 174 from the wound covering 180 to provide the patient with additional mobility.

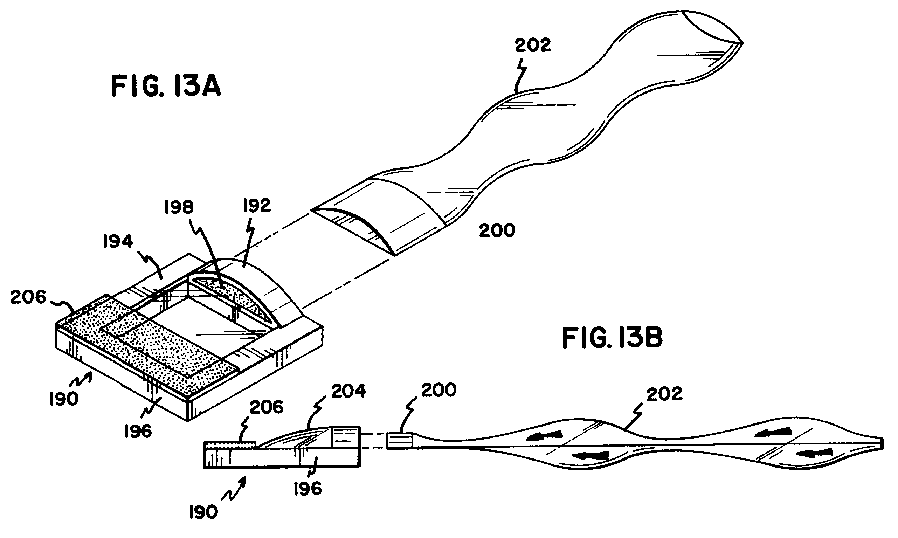

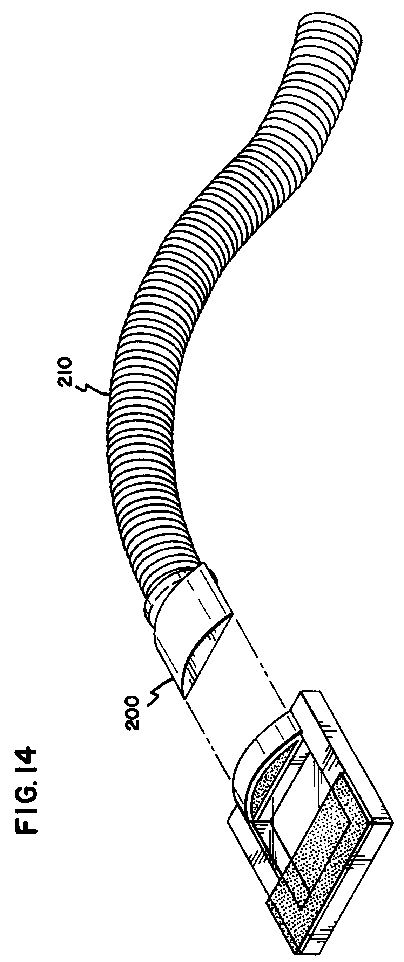

Fig. 13A is a perspective view of an alternate non-contact wound covering 190 having a fluid inlet connector 192 attached to a top surface 194 of the peripheral sealing ring 196. The fluid inlet connector 192 preferably contains an inlet filter media 198. A rigid connector 200 on a fluid inlet line 202 mates with the fluid inlet connector 192. As illustrated in Fig. 13B, a cover 204 extends from the top of the fluid inlet connector 192 across the top of the peripheral sealing ring 196 where it engages with an exhaust filter media 206. Fig. 14 illustrates the embodiment of Figures 13A and 13B utilizing a non-disposable fluid supply line 210.

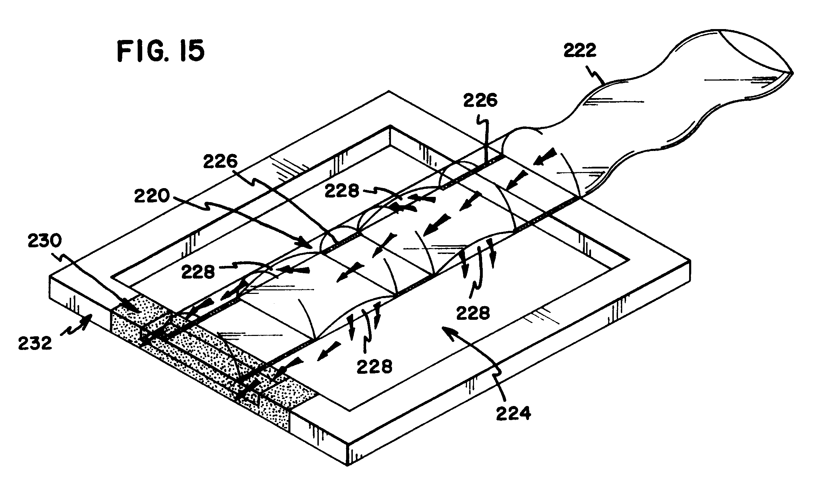

Fig. 15 illustrates an alternate embodiment which utilizes a manifold structure 220 as part of the fluid inlet line 222 to provide even distribution of the fluid being introduced into the treatment volume 224. The fluid inlet line 222 preferably has a series of seals 226 along its edge which are interrupted by a plurality of side openings 228 from which the fluid can be transmitted into the treatment volume 224. The embodiment disclosed in Fig. 15 illustrates an exhaust filter 230 recessed into the side of the peripheral sealing ring 232. However, it will be understood that a variety of exhaust filter configurations are possible with the disclosed manifold structure 220.

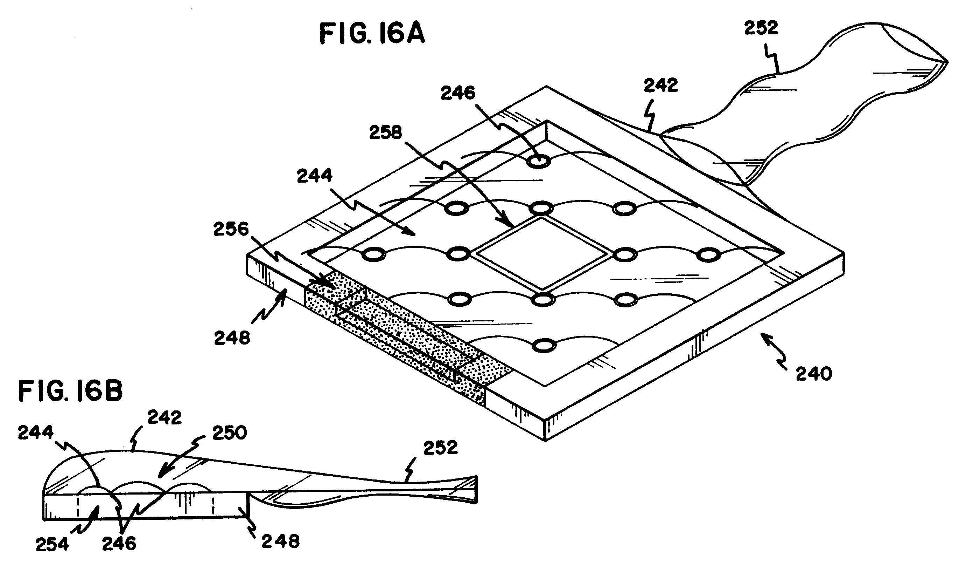

Figs. 16A and 16B illustrate an alternate wound covering 240 with a top barrier layer 242 and a lower layer 244 having a plurality of holes 246. As is illustrated in Fig. 16B, the top cover forms the barrier layer 242 and it extends substantially across the area of the peripheral sealing ring 248. The lower layer 244 likewise extends across the peripheral sealing ring 248. An upper insulating layer 250 is formed between the lower layer 244 and the top of the barrier layer 242. Fluid in the fluid inlet line 252 is directed into the upper insulating layer 250. The pressurized fluid in the upper insulating layer 250 passes through the holes 246 into the treatment volume 254. The holes 246 in the lower layer 244 provide generally even distribution of the fluid within the wound treatment volume 254. An optional seal 258 may be formed in the center portion of the barrier layer 242 and the lower layer 244 to provide the layers with additional structural support. An exhaust filter medium 256 is provided in a recess along one side of the peripheral sealing ring 248 to relieve pressure in the treatment volume 254.

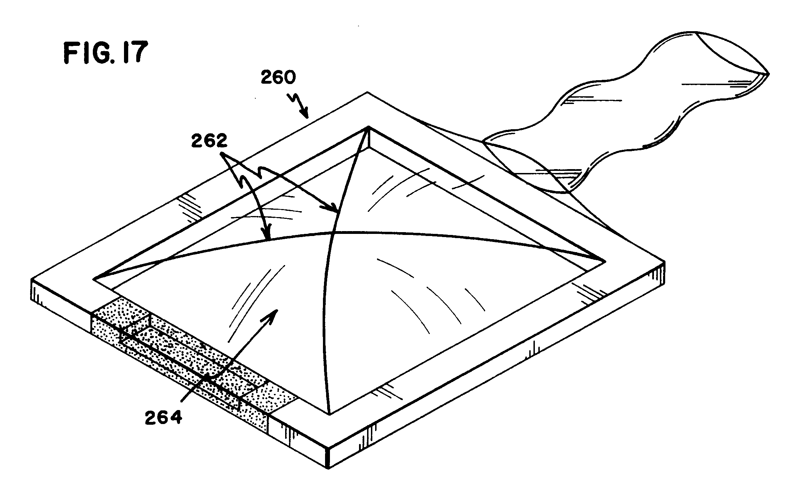

Fig. 17 illustrates an alternate embodiment of a non-contact wound covering 260 utilizing semi-rigid supports 262 to retain the barrier layer 264 above the wound area. It will be understood by those skilled in the art that a variety of semi-rigid supports 262 may be utilized for this application. For example, plastic or resilient rubber materials may provide sufficient support to the barrier layer 264 with a minimum risk of injuring the patient.

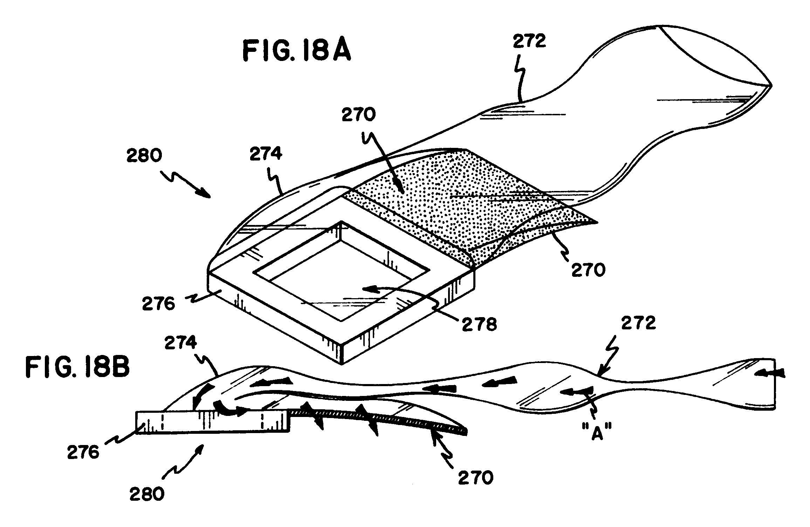

Fig. 18A and Fig. 18B illustrate an alternate exhaust filter medium 270 with an enlarged surface area to accommodate larger volumes of air flow through the non-contact wound covering 280. The exhaust filter is incorporated into the fluid inlet line 272. The fluid inlet line 272 also forms a portion of the barrier layer 274, which is in turn attached to the peripheral sealing ring 276. As is best shown in Fig. 18B, fluid illustrated as the arrows "A" is introduced into the fluid inlet line 272, where it is directed into the wound treatment volume 278, past the wound area and out through the exhaust filter medium 270.

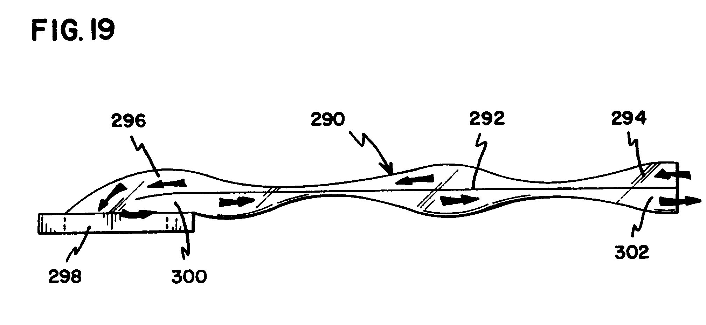

Fig. 19 illustrates a bi-directional line 290 with a center divider 292. Fluid is introduced into the fluid inlet line 294 where it proceeds through a fluid inlet port 296 into the treatment volume 298. The fluid then is forced through a fluid outlet port 300 where it is driven away from the treatment volume 298 in a fluid outlet line 302. It will be understood by those skilled in the art that it would be possible to utilize separate fluid inlet and outlet lines to achieve the same result.

While the invention has been illustrated by means of specific embodiments, it will be evident to those skilled in the art that many variations and modifications may be made therein. However, it is to be understood that the scope of the present invention is to be limited only by the appended claims.

## Claims

1. A wound covering (50) for application to a selected wound area (54) of a patient's body, comprising a peripheral sealing ring (52) with an upper surface (66), a lower surface (72) attachable adjacent said selected wound area (54) with an adhesive (56) and a release liner (74) for attaching the wound covering to the patient's body, and defining a cavity (62), *characterized in that* the wound covering further comprises proximate said upper surface (66) and spaced apart from said selected wound area (54) a heater layer (82, 100) which functions as a barrier layer, or a heater layer (82, 100) in combination with a separate barrier layer (64).

2. The wound covering of claim 1 in which said heater layer comprises a passive heat reflecting layer (82).

3. The wound covering of claim 1 in which said heater layer comprises an active heater layer (100).

4. The wound covering of claim 3 in which said active heater layer includes an electrical resistance heater (106).

5. The wound covering of claim 4 further comprising a pressure activated switch (102) for turning off said active heater layer when pressure is applied to said wound covering.

6. The wound covering of any of claims 3-5 further comprising a thermostat operably coupled to said active heater layer for regulating the temperature of said active heater layer.

7. The wound covering of any of claims 1-6 further comprising a pocket (94) proximate said upper surface (66).

8. The wound covering of any of claims 1-7 in which said barrier layer (64) comprises: a transparent plastic film.

9. The wound covering of any of claims 1-7 wherein said barrier layer (64) comprises: an opaque plastic film.

10. The wound covering of any of claims 1-7 wherein said barrier layer (64) comprises: a gas permeable film.

11. The wound covering of any of claims 1-7 wherein a portion of said barrier layer (64) comprises: a gas permeable filter media.

12. The wound covering of any of claims 1-7 wherein said barrier layer (64) comprises: a polyethylene plastic film.

13. The wound covering of any of claims 1-7 wherein said barrier layer (64) comprises: a polyvinyl chloride plastic film.

14. The wound covering of any of claims 1-13 wherein said peripheral sealing ring (52) comprises: a conformal polymeric foam ring.

15. The wound covering of claim 14 wherein said conformal polymeric foam ring includes a cellular structure forming a reservoir for the storage and release of medicaments.

16. The wound covering of claim 14 wherein said conformal polymeric foam ring includes a cellular structure forming a reservoir for the storage and release of moisture.

17. The wound covering of any of claims 1-16 further comprising: a wound mapping grid proximate said barrier layer (64).

18. A wound covering (50) for application to a selected wound area (54) of a patient's body, comprising a peripheral sealing ring (52) with an upper surface (66), a lower surface (72) attachable adjacent said selected wound area (54) with an adhesive (56) and a release liner (74) for attaching the wound covering to the patient's body, and defining a cavity (62), *characterized in that* the wound covering further comprises a pocket or pocket layer (94) proximate said upper surface (66) and spaced apart from said selected wound area (54), said pocket or pocket layer (94) functioning as a barrier layer or in conjunction with a separate barrier layer (64) and being adaptable to receive a passive heat reflector or an active heater.

19. The wound covering of claim 18 wherein said pocket (94) spans said cavity (62).

20. The wound covering of claim 18 wherein said pocket (94) comprises: a transparent plastic film.

21. The wound covering of claim 18 wherein said pocket (94) comprises: an opaque plastic film.

22. The wound covering of claim 18 wherein said pocket (94) comprises: a gas permeable film.

23. The wound covering of claim 18 wherein a portion of said pocket (94) comprises: a gas permeable filter media.

24. The wound covering of claim 18 wherein said pocket (94) comprises: a polyethylene plastic film.

25. The wound covering of claim 18 wherein said pocket (94) comprises: a polyvinyl chloride plastic film.

26. The wound covering of claim 18 wherein said peripheral ring (52) comprises: a conformal polymeric foam ring.

27. The wound covering of claim 26 wherein said conformal polymeric foam ring includes a cellular structure forming a reservoir for the storage and release of medicaments.

28. The wound covering of claim 26 wherein said conformal polymeric foam ring includes a cellular structure forming a reservoir for the storage and release of moisture.

29. The wound covering of claim 18 further comprising: a wound mapping grid proximate said pocket (94).

30. The wound covering of claim 18 wherein said pocket (94) is adaptable to receive a wound mapping grid.

31. The wound covering of claim 18 wherein said pocket (94) is adaptable to receive a passive heat reflector.

32. The wound covering of claim 18 wherein said pocket (94) is adaptable to receive an active heater.

## Patentansprüche

1. Wundabdeckung (50) zur Anwendung an einem ausgewählten Wundbereich (54) des Körpers eines Patienten, mit einem peripherischen Dichtring (52) mit einer oberen Oberfläche (66), einer unteren Oberfläche (72), die an dem ausgewählten Wundbereich (54) angrenzend mit einem Haftmittel (56) anbringbar ist, und einer ablösbaren Deckschicht (74) zur Befestigung der Wundabdeckung an dem Körper des Patienten, und die einen Hohlraum (62) begrenzt, dadurch gekennzeichnet, daß die Wundabdeckung desweiteren in der Nähe der oberen Oberfläche (66) und von dem ausgewählten Wundbereich (54) beabstandet eine Heizschicht (82, 100), die als Sperrschicht wirkt, oder eine Heizschicht (82, 100) in Kombination mit einer getrennten Sperrschicht (64) aufweist.

2. Wundabdeckung nach Anspruch 1, bei welcher die Heizschicht eine passive, wärmereflektierende Schicht (82) aufweist.

3. Wundabdeckung nach Anspruch 1, bei welcher die Heizschicht eine aktive Heizschicht (100) aufweist.

4. Wundabdeckung nach Anspruch 3, bei welcher die aktive Heizschicht einen elektrischen Widerstandsheizer (106) umfaßt.

5. Wundabdeckung nach Anspruch 4, desweiteren mit einem druckbetätigbaren Schalter (102) zum Abschalten der aktiven Heizschicht wenn auf die Wundabdeckung Druck ausgeübt wird.

6. Wundabdeckung nach einem der Ansprüche 3 bis 5, desweiteren mit einem Thermostaten, der betriebsmäßig gekoppelt ist mit der aktiven Heizschicht zur Regulierung der Temperatur der aktiven Heizschicht.

7. Wundabdeckung nach einem der Ansprüche 1 bis 6, desweiteren mit einer Tasche (94) in der Nähe der oberen Oberfläche (66).

8. Wundabdeckung nach einem der Ansprüche 1 bis 7, bei welcher die Sperrschicht (64) einen transparenten Kunststoffilm umfaßt.

9. Wundabdeckung nach einem der Ansprüche 1 bis 7, bei welcher die Sperrschicht (64) einen opaken Kunststoffilm umfaßt.

10. Wundabdeckung nach einem der Ansprüche 1 bis 7, wobei die Sperrschicht (64) einen gasdurchlässigen Film umfaßt.

11. Wundabdeckung nach einem der Ansprüche 1 bis 7, wobei ein Teil der Sperrschicht (64) ein gasdurchlässiges Filtermedium umfaßt.

12. Wundabdeckung nach einem der Ansprüche 1 bis 7, wobei die Sperrschicht (64) einen Polyethylen-Kunststoffilm umfaßt.

13. Wundabdeckung nach einem der Ansprüche 1 bis 7, wobei die Sperrschicht (64) einen Polvvinylchlorid-Kunststoffilm umfaßt.

14. Wundabdeckung nach einem der Ansprüche 1 bis 13, wobei der peripherische Dichtring (52) einen sich anpassenden, polymeren Schaumstoffring umfaßt.

15. Wundabdeckung nach Anspruch 14, wobei der sich anpassende, polymere Schaumstoffring eine Zell- bzw. Gewebestruktur beinhaltet, welche ein Reservoir für die Speicherung und die Abgabe von Medikamenten bildet.

16. Wundabdeckung nach Anspruch 14, wobei der sich anpassende, polymere Schaumstoffring eine Zell- bzw. Gewebestruktur beinhaltet, welche ein Reservoir für die Speicherung und die Abgabe von Feuchtigkeit bildet.

17. Wundabdeckung nach einem der Ansprüche 1 bis 16, desweiteren mit einem Wundaufzeichnungsgitter in der Nähe der Sperrschicht (64).

18. Wundabdeckung (50) zur Anwendung an einem ausgewählten Wundbereich (54) des Körpers eines Patienten, mit einem peripherischen Dichtring (52) mit einer oberen Oberfläche (66), einer unteren Oberfläche (72), die an dem ausgewählten Wundbereich (54) angrenzend mit einem Haftmittel (56) anbringbar ist, und einer ablösbaren Deckschicht (74) zum Befestigen der Wundabdeckung an dem Körper des Patienten, und die einen Hohlraum (62) begrenzt, dadurch gekennzeichnet, daß die Wundabdeckung desweiteren eine Tasche oder eine Taschenschicht (94) aufweist in der Nähe der oberen Oberfläche (66) und von dem ausgewählten Wundbereich (54) beabstandet, wobei die Tasche oder die Taschenschicht (94) als Sperrschicht oder in Verbindung mit einer getrennten Sperrschicht (64) wirkt und ausgelegt ist, um einen passiven Wärmereflektor oder eine aktive Heizvorrichtung aufzunehmen.

19. Wundabdeckung nach Anspruch 18, wobei die Tasche (94) den Hohlraum (62) überbrückt.

20. Wundabdeckung nach Anspruch 18, wobei die Tasche (94) einen transparenten Kunststoffilm umfaßt.

21. Wundabdeckung nach Anspruch 18, wobei die Tasche (94) einen opaken Kunststoffilm umfaßt.

22. Wundabdeckung nach Anspruch 18, wobei die Tasche (94) einen gasdurchlässigen Film umfaßt.

23. Wundabdeckung nach Anspruch 18, wobei ein Teil der Tasche (94) ein gasdurchlässiges Filtermedium umfaßt.

24. Wundabdeckung nach Anspruch 18, wobei die Tasche (94) einen Polyethylen-Kunststoffilm umfaßt.

25. Wundabdeckung nach Anspruch 18, wobei die Tasche (94) einen Polyvinylchlorid-Kunststoffilm umfaßt.

26. Wundabdeckung nach Anspruch 18, wobei der peripherische Ring (52) einen sich anpassenden, polymeren Schaumstoffring aufweist.

27. Wundabdeckung nach Anspruch 26, wobei der sich anpassende, polymere Schaumstoffring eine Zell- bzw. Gewebestruktur beinhaltet, die ein Reservoir für die Speicherung und die Abgabe von Medikamenten bildet.

28. Wundabdeckung nach Anspruch 26, wobei der sich anpassende, polymere Schaumstoffring eine Zell- bzw. Gewebestruktur beinhaltet, die ein Reservoir für die Speicherung und die Abgabe von Feuchtigkeit bildet.

29. Wundabdeckung nach Anspruch 18, desweiteren mit einem Wundaufzeichnungsgitter in der Nähe der Tasche (94).

30. Wundabdeckung nach Anspruch 18, wobei die Tasche (94) anpaßbar ist, um ein Wundaufzeichnungsgitter aufzunehmen.

31. Wundabdeckung nach Anspruch 18, wobei die Tasche (94) anpaßbar ist, um einen passiven Wärmereflektor aufzunehmen.

32. Wundabdeckung nach Anspruch 18, wobei die Tasche (94) anpaßbar ist, um eine aktive Heizvorrichtung aufzunehmen.

## Revendications

1. Pansement (50) pour plaie, destiné à être appliqué a une zone choisie d'une plaie (54) du corps d'un patient, comprenant un anneau périphérique d'étanchéité (52) ayant une surface supérieure (66), une surface inférieure (72) qui peut être fixée près de la zone choisie de plaie (54) par un adhésif (56), et un revêtement séparable (74), destiné à fixer le pansement au corps du patient et délimitant une cavité (62), caractérisé en ce que le pansement comporte en outre, à proximité de la surface supérieure (66) et à distance de la zone choisie de plaie (54), une couche (82, 100) à organe de chauffage qui joue le rôle d'une couche de barrage, ou une couche à organe de chauffage (82, 100) combinée à une couche séparée de barrage (64).

2. Pansement pour plaie selon la revendication 1, dans lequel la couche à organe de chauffage comporte une couche passive de réflexion de chaleur (82).

3. Pansement pour plaie selon la revendication 1, dans lequel la couche à organe de chauffage comprend une couche à organe actif de chauffage (100).

4. Pansement pour plaie selon la revendication 3, dans lequel la couche à organe actif de chauffage comprend un organe de chauffage par résistance électrique (106).

5. Pansement pour plaie selon la revendication 4, comprenant en outre un interrupteur (102) commandé par une pression et destiné à arrêter le fonctionnement de la couche à organe actif de chauffage lorsqu'une pression est appliquée au pansement.

6. Pansement pour plaie selon l'une quelconque des revendications 3 à 5, comprenant en outre un thermostat couplé fonctionnellement à la couche à organe actif de chauffage pour réguler la température de la couche à organe actif de chauffage.

7. Pansement pour plaie selon l'une quelconque des revendications 1 à 6, comprenant en outre une poche (94) proche de la surface supérieure (66).

8. Pansement pour plaie selon l'une quelconque des revendications 1 à 7, dans lequel la couche de barrage (64) comprend un film de matière plastique transparente.

9. Pansement pour plaie selon l'une quelconque des revendications 1 à 7, dans lequel la couche de barrage (64) comporte un film de matière plastique opaque.

10. Pansement pour plaie selon l'une quelconque des revendications 1 à 7, dans lequel la couche de barrage (64) comporte un film perméable au gaz.

11. Pansement pour plaie selon l'une quelconque des revendications 1 à 7, dans lequel une partie de la couche de barrage (64) comporte un milieu de filtration perméable au gaz.

12. Pansement pour plaie selon l'une quelconque des revendications 1 à 7, dans lequel la couche de barrage (64) comporte un film de polyéthylène.

13. Pansement pour plaie selon l'une quelconque des revendications 1 à 7, dans lequel la couche de barrage (64) comporte un film de chlorure de polyvinyle.

14. Pansement pour plaie selon l'une quelconque des revendications 1 à 13, dans lequel l'anneau périphérique d'étanchéité (52) comporte un anneau de mousse polymère qui s'adapte à la forme.

15. Pansement pour plaie selon la revendication 14, dans lequel l'anneau de mousse polymère qui s'adapte à la forme comprend une structure cellulaire formant un réservoir pour l'accumulation et la libération des médicaments.

16. Pansement pour plaie selon la revendication 14, dans lequel l'anneau de mousse polymère qui s'adapte à la forme a une structure cellulaire formant un réservoir destiné à accumuler et libérer de l'humidité.

17. Pansement pour plaie selon l'une quelconque des revendications 1 à 16, comprenant en outre une grille de dessin de plaie proche de la couche de barrage (64).

18. Pansement (50) pour plaie destiné à être appliqué à une zone choisie de plaie (54) du corps d'un patient, comprenant un anneau périphérique d'étanchéité (52) ayant une surface supérieure (66), une surface inférieure (72) destinée à être fixée près de la zone choisie de plaie (54) par un adhésif (56) et un revêtement séparable (74), pour la fixation du pansement au corps du patient, et délimitant une cavité (62), caractérisé en ce que le pansement pour plaie comporte en outre une poche ou une couche (94) de poche proche de la surface supérieure (66) et placée à distance de la zone choisie de plaie (54), la poche ou la couche de poche (94) jouant le rôle d'une couche de barrage ou coopérant avec une couche séparée de barrage (64) et pouvant être adaptée au logement d'un réflecteur passif de chaleur ou d'un organe actif de chauffage.

19. Pansement pour plaie selon la revendication 18, dans lequel la poche (94) recouvre la cavité (62).

20. Pansement pour plaie selon la revendication 18, dans lequel la poche (94) comporte un film de matière plastique transparente.

21. Pansement pour plaie selon la revendication 18, dans lequel la poche (94) comporte un film de matière plastique opaque.

22. Pansement pour plaie selon la revendication 18, dans lequel la poche (94) comporte un film perméable aux gaz.

23. Pansement pour plaie selon la revendication 18, dans lequel une partie de la poche (94) comprend un milieu de filtration perméable aux gaz.

24. Pansement pour plaie selon la revendication 18, dans lequel la poche (94) comporte un film de polyéthylène.

25. Pansement pour plaie selon la revendication 18, dans lequel la poche (94) comporte un film de chlorure de polyvinyle.

26. Pansement pour plaie selon la revendication 18, dans lequel l'anneau périphérique (52) comporte un anneau de mousse polymère qui s'adapte à la forme.

27. Pansement pour plaie selon la revendication 26, dans lequel l'anneau de mousse polymère qui s'adapte à la forme comprend une structure cellulaire formant un réservoir pour l'accumulation et la libération de médicaments.

28. Pansement pour plaie selon la revendication 26, dans lequel l'anneau de mousse polymère qui s'adapte à la forme comprend une structure cellulaire formant un réservoir pour l'accumulation et la libération d'humidité.

29. Pansement pour plaie selon la revendication 18, comprenant en outre une grille de dessin de plaie proche de la poche (94).

30. Pansement pour plaie selon la revendication 18, dans lequel la poche (94) est destinée à loger une grille de dessin de plaie.

31. Pansement pour plaie selon la revendication 18, dans lequel la poche (94) est destinée à loger un réflecteur passif de chaleur.

32. Pansement pour plaie selon la revendication 18, dans lequel la poche (94) est destinée à loger un organe actif de chauffage.
